# EUROPEAN PATENT SPECIFICATION

(11) **EP 0 530 732 B1**
(45) Date of publication and mention of the grant of the patent: **11.12.1996**
(21) Application number: 92114870.6
(22) Date of filing: 31.08.1992
(51) Int. Cl.: C12Q 1/26, C12Q 1/28

(54) **Method of measuring NADH and NADPH**
Verfahren zur Messung von NADH und NADPH
Méthode pour la mesure de NADH et NADPH

(30) Priority: 06.09.1991 JP 254272/91
(43) Date of publication of application: 10.03.1993
(73) Proprietor: SANWA KAGAKU KENKYUSHO CO., LTD., Nagoya, Aichi-ken (JP)
(72) Inventor: Kurono, Masayasu, c/o Sanwa Kagaku Kenkyusho Co., Nagoya, Aichi-ken (JP); Uno, Shizuo, c/o Sanwa Kagaku Kenkyusho Co. Ltd., Nagoya, Aichi-ken (JP); Takehiro, Osamu, c/o Sanwa Kagaku Kenkyusho Co.Ltd, Nagoya, Aichi-ken (JP); Sawai, Kiichi, c/o Sanwa Kagaku Kenkyusho Co. Ltd., Nagoya, Aichi-ken (JP)
(74) Representative: Wächtershäuser, Günter, Prof. Dr.

(56) References cited:
- EP-A- 0 105 443
- DE-A- 3 725 851
- US-A- 5 091 305
- PATENT ABSTRACTS OF JAPAN vol. 14, no. 14 (C-674)12 January 1990
- PATENT ABSTRACTS OF JAPAN vol. 15, no. 377 (C-870)24 September 1991
- PATENT ABSTRACTS OF JAPAN vol. 10, no. 318 (C-381)29 October 1986

## Description

The present invention relates to a method of measuring β - nicotinamide adenine dinucleotide reduced form (hereinafter referred to as "NADH") or β -nicotinamide adenine dinucleotide phosphate reduced form (hereinafter referred to as "NADPH"), substantially to a method of measuring hydrogen peroxide to be generated through an enzymatic reaction, and can be utilized for clinical examinations to quantitatively measure various biochemical substances or an activity of enzymes in a sample of body fluid such as urine, serum or the like.

At the present time, a measurement of biochemical substances and activity of enzymes has been carried out by using a body fluid as a sample for studying biochemical properties thereof, clinical examinations and the like. In major cases, however, it is difficult to directly and quantitatively measure the substance and then an indirect quantitative measuring method has been carried out whereby an enzyme is reacted with the substance and another substance, quantitatively formed through the enzymatic reaction, is measured.

Exemples of substances to be quantitatively formed or consumed by the enzymatic reaction are (1) hydrogen peroxide as a product of oxidase reaction, and (2) NADH and NADPH as coenzymes relating to a reaction of a dehydrogenase. If such a substance can be measured with high accuracy, a high accuracy measurement of an amount on specific biochemical substance or enzyme activity as objective target will become possible.

As a simple and relatively highly sensitive method through the measurement of hydrogen peroxide to be formed by the reaction of oxidase in said case (1), such a method has generally been carried out with 4-aminoantipyrine (hereinafter referred to as "4-AA") and a phenol or aniline derivative, in the presence of peroxidase, as a color developing reagent of an oxidative coupling reaction. As the phenols, phenol, 2,4-dichlorophenol, 2,4-dibromophenol, 3,5-dichloro-2-hydroxybenzenesulfonic acid and the like can be mentioned. As the aniline derivatives, N,N-dimethylaniline, N,N-diethylaniline, N-ethyl-N-(2-hydroxyethyl)-m-toluidine, N-ethyl-N-(3-methylphenyl)-N'-acetylethylene-diamine, N,N-dimethyl-m-anisidine, N-substituted aniline due to an alkylsulfonic acid or hydroxyalkylsulfonic acid and the like can be mentioned. Further, 3-methyl-2-benzothiazolinone hydrazone (MBTH) may be employed in lieu of 4-AA.

While, there are several methods for measuring NADH and NADPH to be formed by the reaction of dehydrogenase in said case (2), the following ones have been carried out in view of being simple and inexpensive : (a) Method, wherein an absorbance at 340nm of maximum absorption wave-length of NADH and NADPH is directly measured; (b) Method, wherein diaphorase is acted to NADH or NADPH in a sample in the presence of a tetrazolium salt to form a diformazan to colorimetrically measure; and (c) Method, wherein NADH or NADPH is specifically oxidized with NAD(P)H oxidase, as shown in following reaction formula to measure an amount of formed hydrogen peroxide.

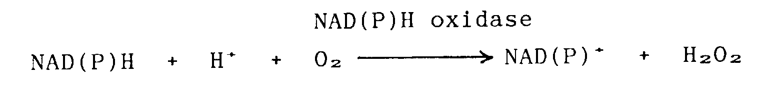

The method (a) is not sufficient in sensitivity, in case of utilizing the same for measuring a trace component in a body fluid sample, since both of NADH and NADPH have only a molecular extinction coefficient of about 6200. The method (b) has the disadvantage that the formazan to be formed is difficult to dissolve in water to cause a pollution in cells.

The method (c) can be carried out by using said color developing reagent of oxidative coupling reaction, for instance as disclosed in Jap. Pat. Nos. Sho 63 (A.D. 1988) - 44882(B) and Hei 1 (A.D. 1989) - 257499(A). However, each of the methods as disclosed in the Japanese official gazettes is not sufficient, in the case where NADH or NADPH should be measured with high accuracy, on the grounds as stated below.

For measuring with high accuracy hydrogen peroxide generated in a sample solution, it is preferable to conduct a chromogenic reaction concurrently with the formation of hydrogen peroxide by the action of NAD(P)H oxidase, since hydrogen peroxide is not so stable. Such an induction method is effective when, particularly, a concentration of substance to be measured is low in the sample. However, the methods disclosed in said official gazettes do not make possible an application of such induction method. According to the disclosures given in the official gazettes, namely, 4-AA and phenol or an aniline derivative are employed as the color developing reagent of oxidative coupling reaction, but with this combination, the color developing substance is reduced by NADH or NADPH in a sample solution and does not develop any color, and thus a color reaction should be carried out, after that NADH or NADPH formed by the enzymatic reaction was completely exhausted by NAD(P)H oxidase. In other words, each of the methods as disclosed in the official gazettes is a 2-stage method and thus can not be seen as an high accuracy method, since there is the possibility that at least a part of the hydrogen peroxide formed by the NAD(P)H oxidase reaction decomposes by reacting with certain component(s) in the sample of body fluid such as serum, urine or the like.

A principal object of the invention is to provide a method to measure NADH or NADPH with high sensitivity and accuracy.

An additional object of the invention is to provide a method of measuring NADH or NADPH with an automatical analyzer.

The inventors have energetically studied and investigated for solving problems in the prior art method of measuring NADH and NADPH, which uses NAD(P)H oxidase, to finally establish the invention.

According to the invention, a method of measuring NADH or NADPH, characterized in that when NADH or NADPH is measured, steps of generating hydrogen peroxide with an action of NAD(P)H oxidase and measuring the hydrogen peroxide by a chromogenic reaction are concurrently carried out. If both steps proceed concurrently, the measuring accuracy of hydrogen peroxide increases, which makes possible the measurement of NADH and NADPH with high sensitivity and accuracy.

When the measurement according to the invention is carried out, there is no restriction of the kind of NAD(P)H oxidase. It should specifically oxidize NADH or NADPH to generate hydrogen peroxide. As a coupler for the color developing reagent, not 4-AA (being widely employed)but MBTH or its derivative is employed, since the latter is not affected by reducing action due to NADH and NAD(P)H. As the MBTH derivatives, those, wherein sulfonyl group, alkyl group, halogen, nitro group, carboxyl group or the like is introduced as a substituent are preferable.

The inventors have carried out measurements of NADH and NADPH according to the method of invention, as given above to find out that a calibration curve shows not a linear curve but nearly a curve of the second order. Such a phenomenon can not be found in the conventional 2-staged method using NAD(P)H oxidase. Therefore, the inventors further studied for elucidating this matter to find out that NAD(P)H forms not only hydrogen peroxide but also an anion of superoxide. Namely, the calibration curve can be made linear by adding superoxide dismutase (hereinafter referred to as "SOD") to coexist in reaction system, when the method according to the invention is carried out.

The SOD is an enzyme which catalytically acts in the reaction shown below and has an action for converting the superoxide anion into hydrogen peroxide. However, the reaction is a relatively speedy one also as non-enzymatical reaction. There is no restriction on the kind of SOD, and those derived from bovine erythrocytes and the like can be employed. In general, SOD shows a sufficient activity in pH range of about 5 - 10 and good stability and thus it is suitable to use the same for the method of invention. It is preferable to set a concentration of SOD in a range of 10 - 200mg/l in the reaction solution.

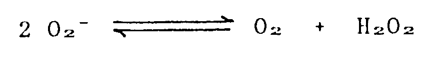

The invention will now be explained, by comparing the same with the conventional method. When serum is selected as a sample to measure 3-hydroxybutyric acid, for instance, the following 3 reactions are utilized in both methods.

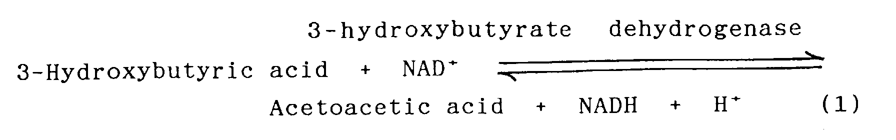

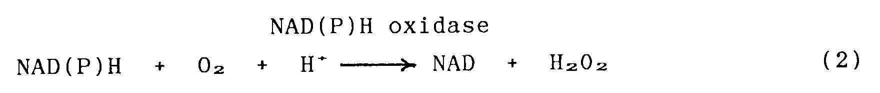

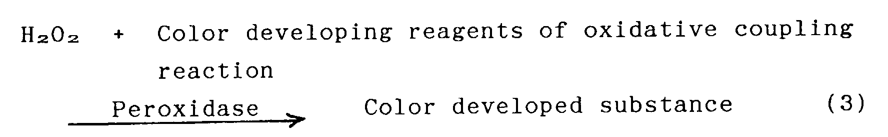

According to the conventional method, the color reaction (3) is carried out, after previously completing the reactions (1) and (2). Therefore, there is the possibility that a part of hydrogen peroxide formed in the reaction (2) reacts with coexisting substance(s) in the serum sample to cause a decomposition thereof. While, according to the method of the invention, the reactions (1), (2) and (3) proceed concurrently, so that hydrogen peroxide, subsequently NADPH or NADPH and further 3-hydroxybutyric acid can be measured with high accuracy. This method according to the invention develops its effectiveness, when the substance to be measured, has an extremely small concentration. Since a single-stage operation is employed for the method of invention, the method is simple. It remarkably reduces the period of time required for the measurement, and scan be applied to a widely employed automatic analyzer to treat samples in large number.

Each of the reagents employed for the method of invention may be in the form of a solution, a powder or the like which is prepared by lyophilization. Since the method of the invention employs only one stage operation all of the reagents may be adsorbed in an absorbing carrier such as a filter paper and the carrier is dried to make a test piece.

The invention will now be further explained in more detail and concretely, with reference to Test Examples and Measuring Example, which shall refer to drawings, wherein
Fig. 1 is a graph showing a relation between a concentration of NADH and absorbance, when SOD co-exists or not; and
Fig. 2 is a graph as a standard calibration curve for measuring 3-hydroxybutyric acid in accordance with the method of invention.

### Test Example 1

(A) Object
Among oxidases, there are those which produce a superoxide anion, for instance as exemplified by xanthine oxidase. Therefore, whether NAD(P)H oxidase and NADH are acted in the presence of nitrotetrazolium blue, and the production of diformazan is measured to examine whether NAD(P)H oxidase has an ability for producing the superoxide anion or not.
(B) Reagents

| | |
|---|---|
| a) Reagent 1 | |
| NADH | 200µM |
| Phosphate buffer (pH 7.5) | 50mM |
| b) Reagent 2 | |
| FAD | 0.1mM |
| Phosphate buffer (pH 7.0) | 50mM |
| FAD : Flavin adenine dinucleotide | |
| c) Reagent 3 | |
| Nitrotetrazolium blue | 1.2mM |
| NAD(P)H oxidase | 2300 U/l |
| Phosphate buffer | 50mM |

(C) Operations
To the reagent 1 (40µl), the reagent 2 (700µl) was added to mix the same and the mixture was incubated for 5 minutes at 37°C. To the resulting solution, the reagent 3 (100µl) was added to cause a reaction for 5 minutes at 37°C to measure an absorbance at 550nm. The absorbance was 0.096, when compensation was carried out with a result of another measurement on a control, which was carried out similarly to the above but using the phosphate buffer solution only, in lieu of the reagent 1. Further, similar operation was carried out by adding 20mg/l of SOD in the reagent 2 to find out that absorbance was 0.013.
From these facts, it was confirmed that NAD(P)H oxidase has an ability of producing not only hydrogen peroxide but also superoxide anion.

### Test Example 2

### (Relation between superoxide anion and calibration curve)

(A) Reagents

| | |
|---|---|
| a) Reagent 1 | |
| MBTH | 0.5mM |
| SOD | 20mg/l |
| FAD | 0.1mM |
| Phosphate buffer (pH 7.0) | 50mM |
| b) Reagent 2 | |
| N-Ethyl-N-sulfopropyl-3,5-dimethylaniline | 5.0mM |
| Peroxidase | 72000 U/l |
| NAD(P)H oxidase | 2300 U/l |
| Phosphate buffer (pH 7.0) | 50mM |

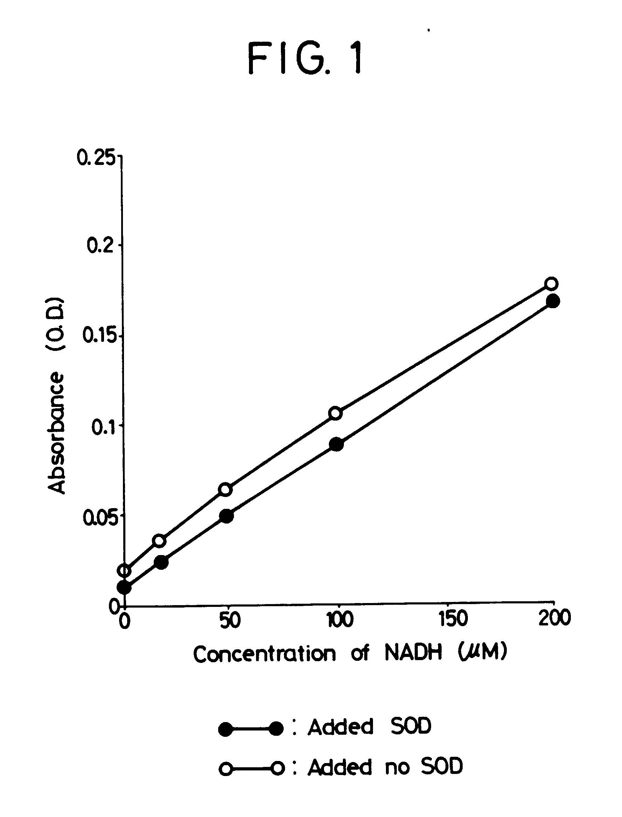

(B) Operations and results
The reagent 1 (350µl) was added to 20µl of NADH solution (0, 20, 50, 100 or 200mM) to incubate for 5 minutes at 37°C. To the resulting solution, the reagent 2 (50µl) was added to cause a reaction at 37°C to measure a change in absorbance per 1 minute at 570nm. Further, similar operations were repeated with use of a reagent similar to reagent 1, but containing no SOD to check a relation between the change in absorbance and concentration of NADH, due to the presence or absence of SOD. Results are shown in Fig. 1. As apparently seen therefrom, no linear relation can be recognized between the concentration of NADH and change in absorbance, when SOD is not contained, but the relation becomes linear by adding SOD to coexist in the reaction system.

### Measuring Example (Measurement of 3-hydroxybutyric acid)

(A) Reagents

| | |
|---|---|
| a) Reagent 1 | |
| NAD⁺ | 0.7mM |
| MBTH | 0.5mM |
| SOD | 20mg/l |
| FAD | 0,1mM |
| Phosphate buffer (pH 7.0) | 50mM |
| b) Reagent 2 | |
| 3-Hydroxybutyrate dehydrogenase | 3320 U/l |
| N-Ethyl-N-sulfopropyl-3,5-dimethylaniline | 50mM |
| Peroxidase | 72000 U/l |
| NAD(P)H oxidase | 2300 U/l |
| Phosphate buffer (pH 7.0) | 50mM |

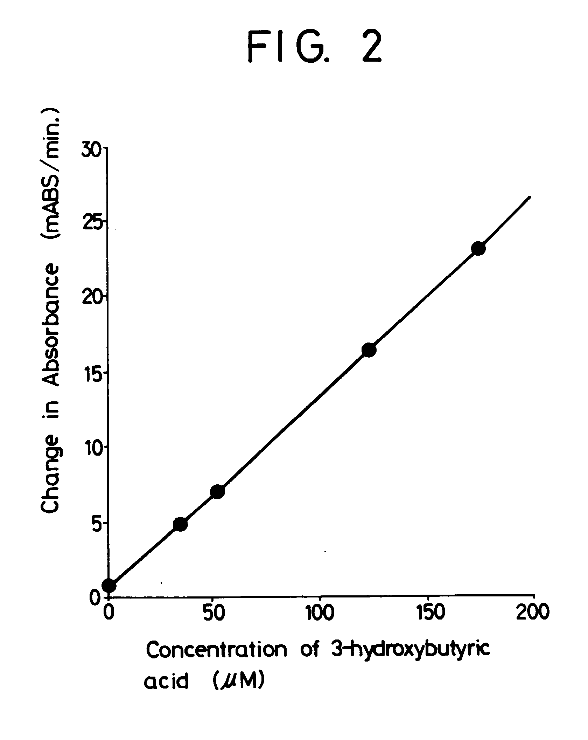

(B) Standard calibration curve
The reagent 1 (350µl) was added to each of various standard 3-hydroxybutyric acid solutions (20µmol) known in concentration thereof to incubate for 5 minutes at 37°C. To the solution, the reagent 2 (50µl) was added to cause a reaction at 37°C to measure a change of absorbance per 1 minute at 570nm and prepare a standard calibration curve from relation between the change of absorbance and concentration of 3-hydroxybutyric acid in the standard solution thereof. The standard calibration curve is shown in Fig. 2.
(C) Measurement of 3-hydroxybutyric acid in serum
Operations similar to those for preparing the standard calibration curve, but by using a sample of serum in lieu of the standard 3-hydroxybutyric acid solution were carried out to measure a change of the absorbance and a level of the change was collated to the standard calibration curve shown in Fig. 2 to determine a concentration of 3-hydroxybutyric acid in the serum sample.
The measurement according to the method as above was repeated 10 times to find that the method shows good reproducibility as the standard deviation is 0.50 and coefficient of variation is 1.93%, near 20µM.
The method according to the invention can be applied for measuring various biochemical substances and activity of enzymes in a body fluid, as exemplified below.

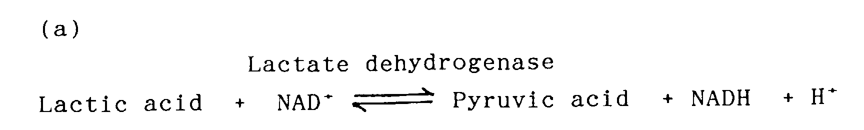

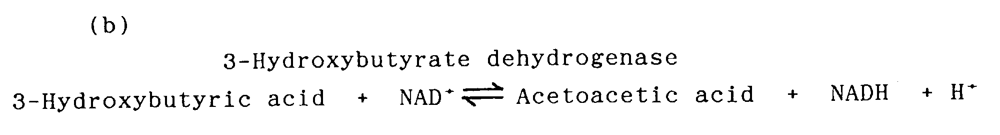

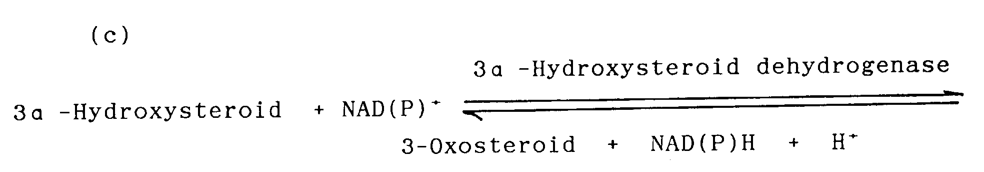

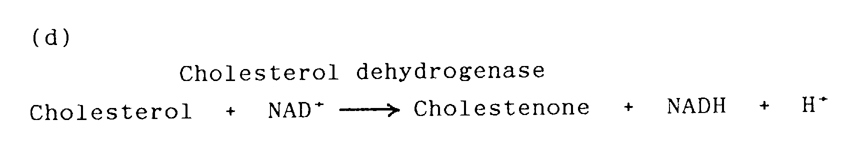

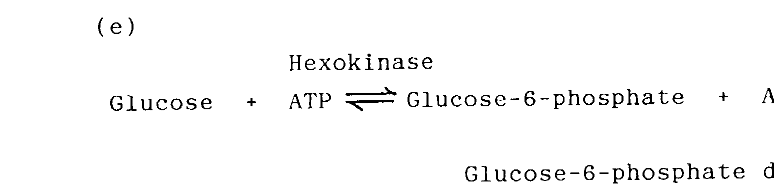

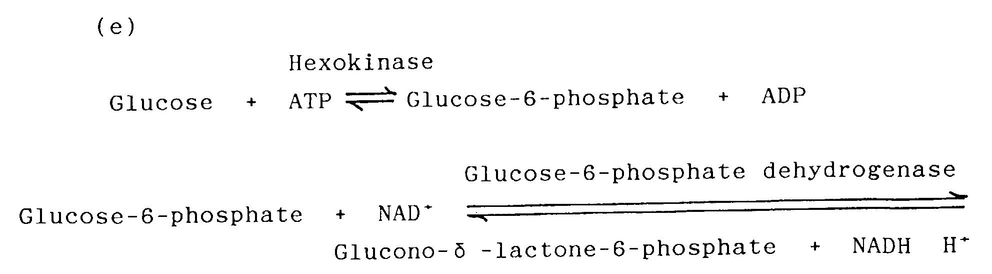

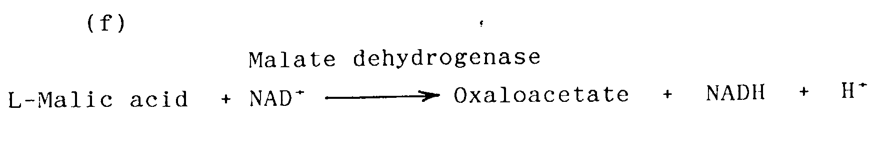

## Claims

1. A method of measuring NADH or NADPH, characterized in that when NADH or NADPH is measured, steps of generating hydrogen peroxide by an action of NAD(P)H oxidase and of measuring the hydrogen peroxide by an oxidative coupling reaction of chromogenic reagents are concurrently carried out, said chromogenic reagents being a combination of peroxidase, a substance selected from aniline and its derivative, and a substance selected from 3-methyl-2-benzothiazolinone hydrazone and its derivative.

2. A method as claimed in Claim 1, wherein an enzyme of superoxide dismutase co-exists in the reaction system.

## Patentansprüche

1. Verfahren zum Messen von NADH oder NADPH, dadurch gekennzeichnet, daß, wenn NADH oder NADPH gemessen wird, Schritte der Erzeugung von Wasserstoffperoxid mittels einer Einwirkung von NAD(P)H-Oxidase und des Messens des Wasserstoffperoxids durch eine oxidative Kupplungsreaktion von chromogenen Reagenzien gleichzeitig durchgeführt werden, wobei die chromogenen Reagenzien eine Kombination von Peroxidase, einer Substanz, die aus Anilin und dessen Derivaten ausgewählt ist, und einer Substanz ist, die aus 3-Methyl-2-benzothiazolinonhydrazon und dessen Derivaten ausgewählt ist.

2. Verfahren nach Anspruch 1, in dem ein Superoxiddismutase-Enzym im Reaktionssystem mit vorliegt.

## Revendications

1. Procédé pour la mesure de NADH et de NADPH, caractérisé en ce que lorsque l'on mesure le NADH ou le NADPH, on effectue simultanément les étapes de production de peroxyde d'hydrogène par l'action d'une NAD(P)H oxydase et de mesure du peroxyde d'hydrogène par une réaction de couplage oxydatif de réactifs chromogènes, lesdits réactifs chromogènes étant une combinaison d'une peroxydase, d'une substance choisie parmi l'aniline et ses dérivés et d'une substance choisie parmi la 3-méthyl-2-benzothiazolinone hydrazone et ses dérivés.

2. Procédé selon la revendication 1, dans lequel une enzyme dismutase de superoxyde coexiste dans le système de réaction.
